(19) Europäisches Patentamt
European Patent Office
Office européen des brevets

(11) EP 3 271 325 B1

(12) EUROPEAN PATENT SPECIFICATION

(45) Date of publication and mention of the grant of the patent:
**02.12.2020 Bulletin 2020/49**

(21) Application number: **15778398.6**

(22) Date of filing: **01.09.2015**

(51) Int Cl.:
***C07C 213/02*** *(2006.01)* ***C07C 217/08*** *(2006.01)*
***C07C 231/02*** *(2006.01)* ***C07C 233/18*** *(2006.01)*
***B01J 31/18*** *(2006.01)* ***B01J 31/22*** *(2006.01)*

(86) International application number:
**PCT/PT2015/000044**

(87) International publication number:
**WO 2016/153374 (29.09.2016 Gazette 2016/39)**

# (54) PROCESS OF PRODUCTION OF (S) -METOLACHLOR

VERFAHREN ZUR HERSTELLUNG VON (S)-METOLACHLOR

PROCÉDÉ DE PRODUCTION DE (S)-MÉTOLACHLORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.03.2015 PT 2015108303**

(43) Date of publication of application:
**24.01.2018 Bulletin 2018/04**

(73) Proprietor: **Ascenza Agro, S.A.**
**1990-207 Lisboa (PT)**

(72) Inventors:
- **TRINDADE SANTOS NEVES, Jose Fernando**
  **2910-442 - Setúbal (PT)**
- **FLORENCIO NOGUEIRA, José Manuel**
  **1749-016 - Lisboa (PT)**
- **ISMAIL AHMAD, Samir Marcos**
  **1749016 - Lisboa (PT)**
- **BATHIA, Surendra**
  **400604 Maharashtra (IN)**
- **GRAINGER, Damian**
  **Cambridge CB4 0FP (GB)**
- **ZANOTTI GEROSA, Damian**
  **Cambridge CB4 0FP (GB)**

(56) References cited:
**WO-A1-95/21151 WO-A1-2007/017522**
**CN-A- 1 768 944**

- **BLASER* H-U ET AL: "Tunable ferrocenyl diphosphine ligands for the Ir-catalyzed enantioselective hydrogenation of N-aryl imines", JOURNAL OF ORGANOMETALLIC CHEMISTRY, ELSEVIER-SEQUOIA S.A. LAUSANNE, CH, vol. 621, no. 1-2, 1 March 2001 (2001-03-01), pages 34-38, XP004231234, ISSN: 0022-328X, DOI: 10.1016/S0022-328X(00)00766-X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 271 325 B1

## Description

### FIELD OF THE INVENTION

The present invention relates to a process of production of the herbicide *(S)*-metolachlor comprising a step of hydrogenation of *N*-(2'-methyl-6'-ethyl-phenyl)-*N*-(1-methoxymethyl)ethylimine to *(S)*-*N*-(2'-methyl-6'-ethyl-phenyl)-*N*-(1-methoxylmethyl)ethylamine in the presence of an iridium complex and a phosphine-phosphoramidite ligand.

### BACKGROUND OF THE INVENTION

The synthesis of *(S)*-metolachlor is described by the following sequence of reactions:

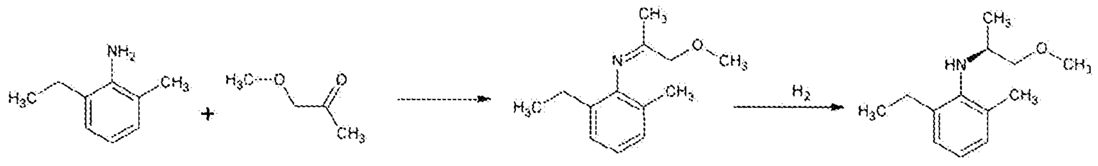


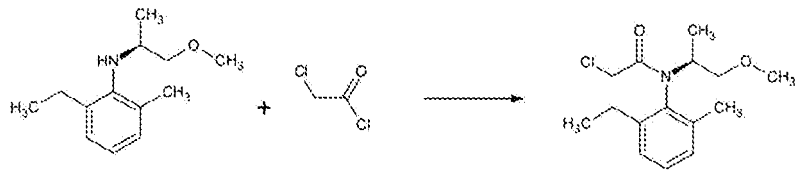

(S)-metolachlor

The key step in this synthesis is the asymmetric hydrogenation of *N*-(2'-methyl-6'-ethyl-phenyl)-*N*-(1-methoxymethyl)ethyl imine (I) to (*S*)-*N*-(2'-methyl-6'-ethyl-phenyl)-*N*-(1-methoxylmethyl)ethylamine (II):

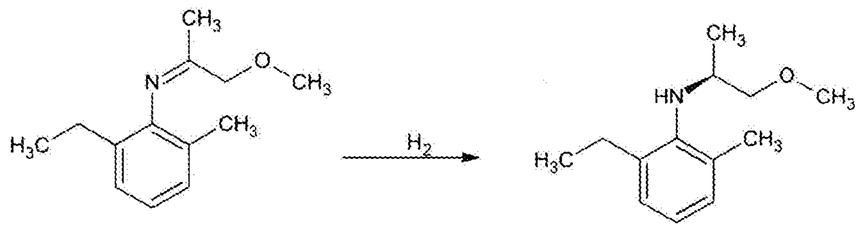


(I) (II)

The first successful catalyst for this transformation was disclosed in patent application WO95/21151. The catalyst has the general formula [XIrYZ], wherein:

- Y is a ferrocenyl-diphosphine ligand of the formula (III), also known as Xyliphos,

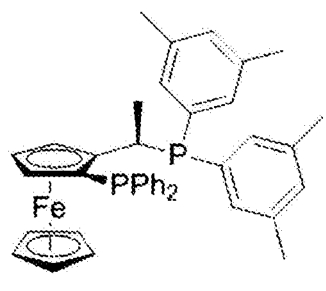

(III)

wherein:

- Ph is phenyl,
- X is a diene ligand, more specifically 1,5-cyclooctadiene (COD), and
- Z is a halogen, more specifically chlorine.

The reaction can be carried out with or without an inert solvent, and the reaction mixture contained an ammonium chloride, bromide or iodide and additionally an acid. The asymmetric hydrogenation of (I) was described in detail in example 1: the reaction was carried out at a temperature of 50 °C and at a $H_2$ pressure of 80 bar, in the presence of tetrabutylammonium iodide and acetic acid, without any other solvent; the molar ratio between the ligand and the substrate (I) was 0.0135 mmol : 1 mol (1mol of ligand : 74000 mol of substrate). After 18 hours, the conversion was 97% and the optical yield was 75.6% (S-isomer).

Patent US6525210 discloses a chiral ligand with binaphtyl motif connected by a ferrocene backbone (IV):

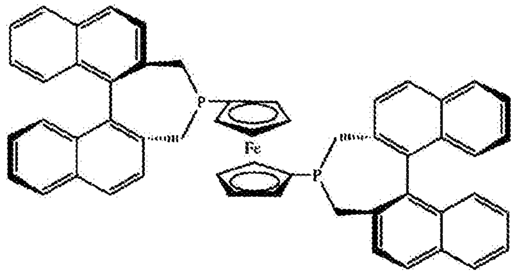

(IV)

The patent description further provides a general process for the hydrogenation of imines: the reaction was carried out in dichloromethane, at room temperature and a hydrogen pressure of 68 bar and the ligand to substrate ratio was 0.044 mmol : 1 mol (1 mol of ligand : 22700 mol of substrate). An enantiomeric excess of 80% of the (S)-isomer (II) was obtained when the above conditions were used for the hydrogenation of (I).

Patent application WO2010094164 discloses a ligand with the general formula (V):

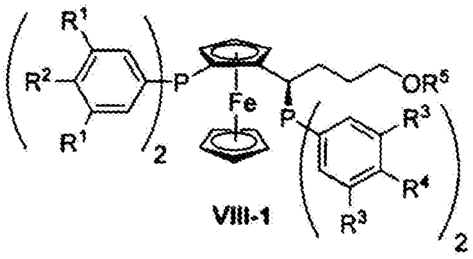

(V)

The asymmetric hydrogenation of (I) was carried out using a ligand (V) wherein $R^1$, $R^2$ and $R^5$ are H, $R^3$ is $CH_3$ and $R^4$ is $N(CH_3)_2$, and the reaction was carried out in tetrahydrofuran, at 50 °C and a hydrogen pressure of 80 bar, in the presence of 2,3,4,5,6-pentafluorobenzyl bromide, at a molar ratio of ligand : substrate of 0.012 mmol : 1 mol (1 mol of ligand : 83000 mol of substrate). After 7 hours, the conversion was >99% and an enantiomeric excess of 78% (S-isomer) was obtained.

Patent application CN101857612 discloses a ligand with the general formula (VI);

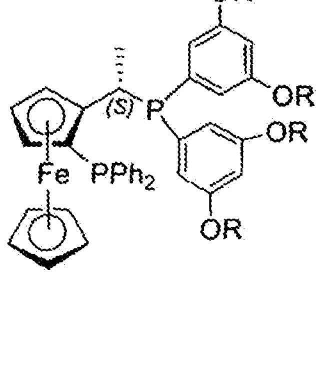

(VI)

wherein Ph is phenyl and R is methyl (VIa) or tert-butyl (VIb). When (VIa) was used at a molar ratio of metal to ligand of 1:1 and a substrate to metal ratio of 100000:1, after 10 hours the conversion was 98% and the enantiomeric excess was 83%; when the substrate to metal ratio was increased to 200000:1 it took 15 hours to achieve a conversion of 98%, but the enantiomeric excess decreased to 80%. Replacing ligand (VIa) by (VIb), a conversion of 98% and an enantiomeric excess of 86.5% were obtained at a substrate to metal ratio of only 50000:1 and after 26 hours. When the reaction temperature was increased from 40 °C to 115 °C, the time required to achieve the same conversion was 15 hours but the enantiomeric excess was reduced to 80%.

Patent application WO2012116493 discloses a chiral ligand with the general formula (VII);

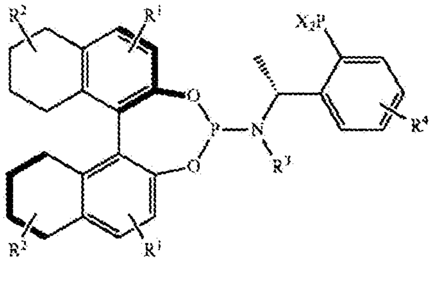

(VII)

The asymmetric hydrogenation of (I) was carried out using a ligand (VII) wherein $R^1$, $R^2$, $R^3$ and $R^4$ are H and X is phenyl, at a temperature of 50 °C and at a $H_2$ pressure of 80 bar, in the presence of tetrabutylammonium iodide and dichloroethane as the solvent. No acid was required and the molar ratio between the ligand and the substrate was 0.0083 mmol : 1 mol (1 mol ligand: 120000 mol substrate). After 18 hours, the conversion was 97% and the optical yield 93.5% (S). No information about the enantiomeric excess is provided.

Hu, Xiang-Ping et al., Organic Letters, 2012, Vol.14, No13, 3554-3557*,* reported that ligand (VII) was used for the asymmetric hydrogenation of compound (I). The hydrogenation was performed at 100 °C for 18 hours at a $H_2$ pressure of 80 bar with $Bu_4NI$ as the additive, employing a substrate to catalyst loading of 100000:1. The conversion was complete and the enantiomeric excess was 80%.

Novel phosphine-phosphoramidite compounds with the formula (VIIIa) or (VIIIb) were disclosed in WO2006012045A1:

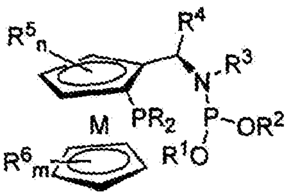

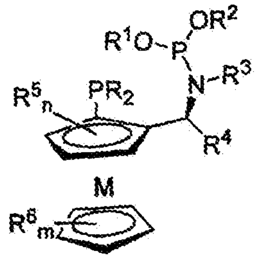

(VIIIa) (VIIIb)

Especially preferred were the compounds wherein R is aryl, most preferably phenyl, $R^1$ is aryl, most preferably phenyl, 4-methoxyphenyl, or 4-trifluoromethylphenyl; $R^2$ is aryl, most preferably phenyl, 4-methoxyphenyl, or 4-trifluoromethylphenyl, or $R^1$ and $R^2$ are collectively 1,2-ethanediyl, 1,3-propanediyl, 1,2-benzenediyl, 2,2'-biphenyldiyl, racemic 1,1'-binaphthyl-2,2'-diyl, (R,R)-1,1'-binaphthyl-2,2'-diyl, or (S,S)-1,1'-binaphthyl-2,2'-diyl; $R^3$ is hydrogen, $C_1$-$C_6$ alkyl, or aryl, most preferably methyl; $R^4$ is hydrogen or $C_1$-$C_6$ alkyl, most preferably methyl; $R^5$ and $R^6$ are hydrogen; n is 0; m is 0; and M is iron. The patent application shows the use of this ligand with a catalytically-active metal selected from rhodium, iridium, or ruthenium, preferably ruthenium, for the asymmetric hydrogenation of an enamide of the general formula (IX) to the corresponding amino acid derivative (X):

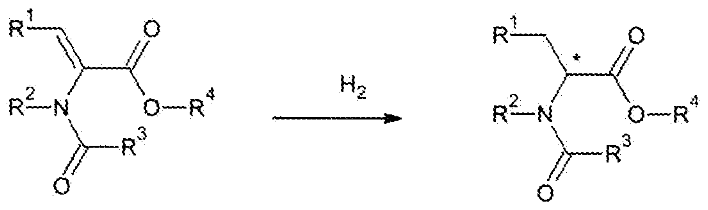

(IX) (X)

However, no reference or suggestion is made to the use of the above ligand for the catalytic asymmetric hydrogenation of compound (I).

Hu, Xiang-Ping et al., Organic Letters, 2004, Vol.6, No20, 3585-3588, also reported that ligand (XI)

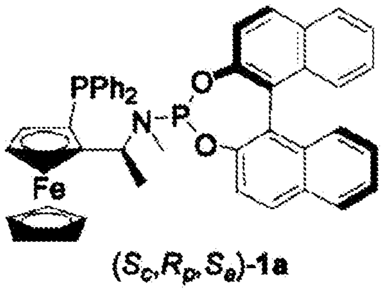

$(S_c,R_p,S_a)$-1a (XI)

was used for the rhodium catalyzed asymmetric hydrogenation of enamides of general formula (XII)

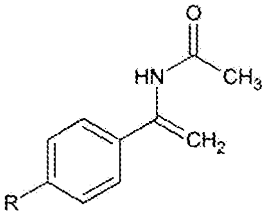

R= H, F, Cl, Br, $CF_3$ (XII),

and also of dimethyl Itaconate and methyl (Z)-acetamidocinnamate. In all cases the ligand was completed with $Rh(COD)_2BF_4$.

No reference is made to the combination of this ligand with Iridium catalysts or to the asymmetric hydrogenation of compound (I).

**DETAILED DESCRIPTION OF THE INVENTION**

The object of the invention is a process for the production of (*S*)-metolachlor comprising a step of asymmetric hydrogenation of *N*-(2'-methyl-6'-ethyl-phenyl)-*N*-(1-methoxymethyl)ethylimine (I) to (*S*)-*N*-(2'-methyl-6'-ethyl-phenyl)-*N*-(1-methoxylmethyl)ethylamine (II) having the following characteristics:

- High conversion;
- High selectivity, measured by the enantiomeric excess of the S-isomer;
- Low catalyst to substrate ratio;
- Acceptable reaction time.

Surprisingly, it was found that when *N*-(2'-methyl-6'-ethyl-phenyl)-*N*-(1-methoxylmethyl)ethylimine (I) contacts with hydrogen in the presence of:

- a coordination compound formed by a ligand having the structural formula (XVII) and (XVIII) of table I (see below). and a diiridium complex of the formula $[IrXCl]_2$, wherein X is 1,5-cyclooctadiene (COD), at a molar ratio of ligand :

Iridium in the range of 1:2 to 2:1;
- an additive selected from iodine or an alkaline metal salt thereof; and
- an aprotic solvent,

at a temperature in the range of 20 °C to 90 °C and a hydrogen pressure in the range of 5 to 80 bar, a conversion to (*S*)-*N*-(2'-methyl-6'-ethyl-phenyl)-*N*-(1-methoxylmethyl) ethylamine (II) greater than 95% and an enantiomeric excess of the S-isomer greater than 81% are obtained.

A series of ligands is presented in table I. Only ligands of formula (XVII) and (XVIII) are considered to be part of the present invention.

Table I

| No | Name | Structure |
|---|---|---|
| XIV | (*R*) -*N*-[ (*R*), (*R*)-1, 1'-binaphthyl-2, 2'-dioxyphosphino]-*N*-methyl-1-[ (*S*)-2-(diphenylphosphino)ferrocenyl]ethylamine | 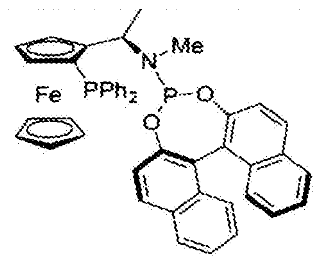 |
| XV | (*R*) -*N*-[(*S*),(*S*)-1,1'-binaphthyl-2,2'-dioxyphosphino]-*N*-methyl-1-[ (*S*)-2-(diphenylphosphino)ferrocenyl]ethylamime | 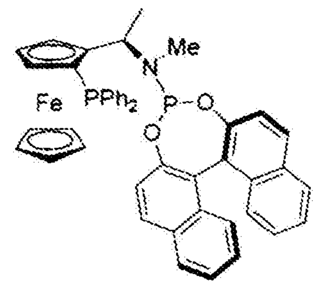 |
| XVI | (*R*)-*N*-[(*S*),(*S*)-1,1'-binaphthyl-2,2'-dioxyphosphino]-1-[(*S*)-2-(diphanylphosphino)ferrecenyl]ethylamine | 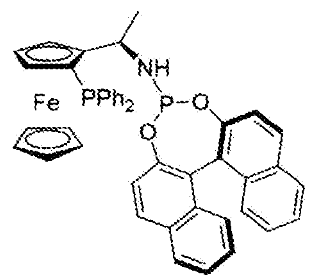 |
| XVII | (*R*)-*N*-[(*R*),(*R*)-5,5',6,6',7,7',8,8'-octahydro-1,1'-binaphthyl-2,2'-dioxyphoaphino]-*N*-methyl-1-[ (*S*)-2-(diphenylphosphino)ferrocenyl] ethylamine | 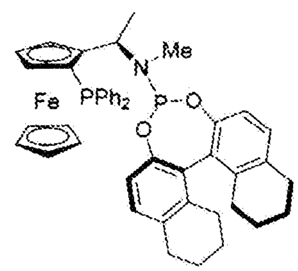 |

(continued)

| No | Name | Structure |
|---|---|---|
| XVIII | (*R*)-*N*-[(*R*),(*R*)-5,5',6,6',7,7',8,8'-octahydro-1,1'-binaphthyl-2,2'-dioxyphosphino1-1-[(*S*)-2-(diphenylphosphino)ferrocenyl]ethylamine | 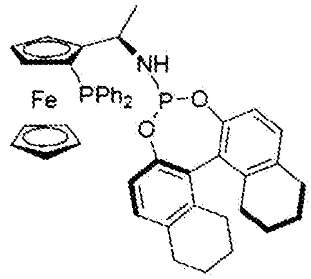 |
| XIX | (*R*)-*N*-[3,3',5,5'-tetramethyl-1,1'-biphenyl-2,2'-dioxyphosphino]-*N*-methyl-1-[(*S*)-2-(diphenylphosphino)ferrocenyl] ethylamine | 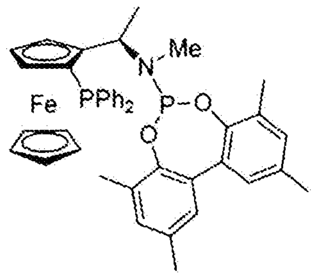 |
| XX | (*R*)-*N*-[3,3',5,5'-tetra-*tert*-butyl-1,1'-bighenyl-2,2'-dioxyphosphino]-*N*-methyl-1-[(*S*)-2-(diphenylphosphino)ferrocenyl] ethylamine | 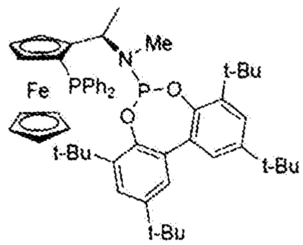 |
| XXI | (*R*)-*N*-[(*R*),(*R*)-1,1'-binaphthyl-2,2'-dioxyphosphino]-*N*-methyl-1-[(*S*)-2-(bis(3,5-dimethylphenyl)phosphino) ferrocenyl]ethylamine | 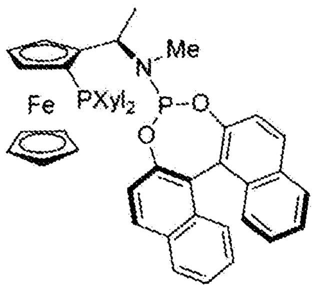 |
| XXII | (*R*)-*N*-[(*R*),(*R*)-5,5',6,6',7,'7',8,8'-octahydro-1,1'-binaphthyl-2,2'-dioxyphosphiftol-*N*-methyl-1-[(*S*)-2-(bis(3,5-dimthylphenyl)phosphino) ferrocenyl]ethylamine | 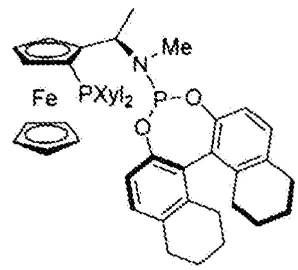 |

The molar ratio between the ligand (L) and the metal (Ir) is in the range of 2:1 to 1:2 (L:Ir). A molar ratio of 1.1:1 (L:Ir) is particularly preferred.

The molar ratio between the ligand and the imine (I) is in the range of 100:1 to 300000:1, preferably from 100000:1 to 250000:1, most preferably from 200000:1 to 250000:1.

The reaction is carried out in the presence of an aprotic solvent selected from the group consisting of dichloromethane, toluene, xylene and n-heptane, preferably toluene.

The ratio between the solvent and the imine (I) is in the range of 1:2 (volume:weight) to 150:1 (volume:weight), preferably 1:1 (volume:weight) to 4:1 (volume:weight), most preferably 1:1 (volume:weight).
The additive is iodine or an alkaline metal salt thereof selected from the group consisting of LiI, NaI and KI, preferably iodine or LiI.
The molar ratio between the additive and the imine (I) is in the range of 0.00125:1 to 0.05:1, preferably 0.0025:1 to 0.005:1.
The reaction temperature is in the range of 20 °C to 90 °C, preferably in the range of 50 °C to 70 °C, most preferably 70 °C.
The hydrogen pressure is in the range of 5 to 80 bar, preferably in the range of 20 to 80 bar, most preferably 30 bar.

## Examples

The following embodiments illustrate the process according to the invention.
In the following examples, the conversion is calculated using the results of the HPLC analysis of the reaction product and equation (I) :

$$\% \text{ conversion} = (AS+AR)/(AS+AR+AI+AA)*100 \quad (I)$$

wherein:

AS- HPLC area of the peak corresponding to the (S)-isomer of the amine (II)
AR- HPLC area of the peak corresponding to the (R)-isomer of the amine (II)
AI- HPLC area of the peak corresponding to the imine (I)
AA - HPLC area of the peak corresponding to aniline (precursor of the imine (I)).

The enantiomeric excess is the difference between the area percentages of the (S) and (R) isomers of the amine (II).
In the following examples, unless specifically indicated, the ratios are per mol.

## Example 1

A reaction vial is charged with ligand, $(IrCODCl)_2$ (ligand:Ir ratio of 1:1) and the solvent (0.1 ml) and stirred under nitrogen for 30 minutes. To the vial is added a solution of the imine (I) (0.1 mmol) in the solvent (0.1 ml) and a solution of the additive (0.005 mmol) in the solvent (0.1 ml). The Ir: imine ratio is 1:100 and the additive:imine ratio is 0.05:1. The reaction vial is loaded into the hydrogenation equipment, which is purged with nitrogen. The vessel is then purged with $H_2$ in three cycles (10-20 bar, normal pressure) with stirring (750 rpm). The hydrogen pressure is set at 20 bar and the reaction is carried out at 20 °C for 16 h under stirring (750 rpm). After cooling to room temperature the pressure is released, reaction mass is sampled (~10 µl, diluted to 1.5 ml with isopropanol) and analysed by HPLC. The following values of conversion and enantiomeric excess (% e.e.) were obtained (all tests made on ligands other than XVII and XVIII are comparatives examples):

Table II

| Ligand | Conversion (%) | e.e. (%) | Solvent | Additive |
|---|---|---|---|---|
| Compound XIV | 97 | 83 | Dichloromethane | $I_2$ |
| Compound XV | 95 | 81 | Dichloromethane | $I_2$ |
| Compound XVI | 95 | 81 | Dichloromethane | $I_2$ |
| Compound XVII | 97 | 93 | Dichloromethane | $I_2$ |
| Compound XVIII | 96 | 93 | Dichloromethane | $I_2$ |
| Compound XIX | 95 | 87 | Dichloromethane | $I_2$ |
| Compound XX | 95 | 87 | Dichloromethane | $I_2$ |
| Compound XXI | 95 | 83 | Dichloromethane | $I_2$ |
| Compound XXII | 95 | 83 | Dichloromethane | $I_2$ |

(continued)

| Ligand | Conversion (%) | e.e. (%) | Solvent | Additive |
|---|---|---|---|---|
| Compound XVII | 97 | 92 | Dichloromethane | NaI |
| Compound XVII | 97 | 81 | Dichloromethane | KI |
| Compound XVII | 97 | 92 | Toluene | $I_2$ |
| Compound XVII | 97 | 93 | Toluene | NaI |

**Example 2**

2.3 mg of ligand (XVIII) and 1 mg of $[IrCODCl]_2$ (ligand:Ir ratio of 1.1:1) were pre-mixed in 0.25 ml of toluene under nitrogen and the mixture was stirred for 15-20 minutes. 50 μl of the previous mixture were fed to a reaction vial containing 32.6 mg LiI (LiI:imine ratio of 0.02:1), 2.5 g of imine (I) (Ir:imine ratio of 1:20000) and 2.5 ml of toluene (toluene:imine ratio of 1:1 volume:weight), and the reaction vial was loaded into the hydrogenation equipment, which was purged with nitrogen. The reaction vessel was purged with $H_2$ in five cycles (20-30 bar, normal pressure) under stirring (650 rpm). The hydrogen pressure was set as indicated in table III and the mixture heated to 70 °C for 20 hours under stirring (650 rpm). After cooling to room temperature, the pressure was released, reaction mass was sampled (~10 μl, diluted to 1.5 ml with isopropanol) and analysed by HPLC. The following values of conversion and enantiomeric excess were obtained:

Table III

| Hydrogen pressure (bar) | % conversion | % enantiomeric excess (S isomer) |
|---|---|---|
| 5 | 97,5 | 90,5 |
| 15 | 98,1 | 90,4 |
| 20 | 97,8 | 89,5 |
| 25 | 98,2 | 90,0 |
| 30 | 97,7 | 90,4 |

**Example 3**

2.3 mg of ligand (XVIII) and 1 mg of $[IrCODCl]_2$ (ligand:Ir ratio of 1.1:1) were pre-mixed in 1 ml of toluene under nitrogen and the mixture was stirred for 20-30 minutes. 20 μl of the previous mixture were fed to a reaction vial containing 7.7 mg $I_2$ ($I_2$:imine ratio of 0.005:1), 1.25 g of imine (I) (Ir:imine ratio of 1:100000) and 1.25 ml of toluene (toluene:imine ratio of 1:1 volume:weight), and the reaction vial was loaded into the hydrogenation equipment, which was purged with nitrogen. The reaction vessel was purged with $H_2$ in five cycles (20-30 bar, normal pressure) under stirring (650 rpm). The hydrogen pressure was set at 30 bar and the mixture heated to 70 °C for 16 hours under stirring (650 rpm). After coaling to room temperature, the pressure was released, reaction mass was sampled (~10 μl, diluted to 1.5 ml with isopropanol) and analysed by HPLC. The conversion of the imine (I) to (*S*)-*N*-(2'-methyl-6'-ethyl-phenyl)-*N*-(1-methoxylmethyl)ethylamine (II) was 98.8% and the enantiomeric excess of the (*S*)-isomer was 88.3%. The estimated reaction time, based on hydrogen uptake data, was 2 hours.

**Examples 4 to 6**

Using the procedure of example 3, but replacing $I_2$ with LiI, the following values of conversion and enantiomeric excess were obtained:

Table IV

| | Example 4 | Example 5 | Example 6 |
|---|---|---|---|
| LiI:imine ratio | 0.005:1 | 0.005:1 | 0.005:1 |
| Toluene:imine ratio (volume:weight) | 1:1 | 1:1. | 1:1 |
| Temperature (°C) | 70 | 60 | 80 |

(continued)

| | Example 4 | Example 5 | Example 6 |
|---|---|---|---|
| $H_2$ Pressure (bar) | 30 | 30 | 30 |
| Estimated reaction time (hours) | 2.5 | 6.0 | 1.5 |
| % conversion | 98.3 | 97.7 | 98.9 |
| % enantiomeric excess | 88.4 | 89.3 | 87.5 |

**Examples 7 to 9**

Using the procedure of examples 4 to 6, but replacing toluene with n-heptane, *m*-xylene and *o*-xylene, the following values of conversion and enantiomeric excess were obtained:

Table V

| | Example 7 | Example 8 | Example 9 |
|---|---|---|---|
| n-heptane:imine ratio (volume:weight) | 1:1 | --- | --- |
| *m*-xylene:imine ratio (volume:weight) | --- | 1:1 | --- |
| *o*-xylene: imine ratio (volume:weight) | --- | --- | 1: 1 |
| Temperature (°C) | 70 | 70 | 70 |
| $H_2$ Pressure (bar) | 30 | 30 | 30 |
| Estimated reaction time (hours) | 3.5 | 3.0 | 3.5 |
| % conversion | 98.6 | 97.7 | 97.7 |
| % enantiomeric excess | 89.1 | 88.4 | 88.5 |

**Example 10**

As in example 5, but with a Ir:ligand ratio of 2:1. The conversion was 97.0% and the enantiomeric excess 88.8% (S-isomer). The estimated reaction time, based on hydrogen uptake data, was 15 hours.

**Example 11**

2.2 mg of ligand (XVIII) and 1 mg of $[IrCODCl)_2$. (ligand:Ir ratio of 1:1) were pre-mixed in 1 ml of toluene under nitrogen and the mixture was stirred for 30 minutes, 80 μl of the previous mixture were fed to a reaction vessel containing 16.3 mg LiI (LiI:imine ratio of 0.005:1), 5 g of imine (I) (Ir:imine ratio of 1:100000) and 5 ml of toluene (toluene:imine ratio of 1:1 volume:weight), and the reaction vessel was purged with nitrogen. The reaction vessel was purged with $H_2$ in three cycles (10-20 bar, normal pressure) under stirring (750 rpm). The hydrogen pressure was set at 80 bar and the mixture heated to 60 °C for 20 hours under stirring (750 rpm). After cooling to room temperature the pressure was released, reaction mass was sampled (~10 μl, diluted to 1.5 ml with isopropanol) and analysed by HPLC. The conversion of the imine (I) to (*S*)-*N*-(2'-methyl-6'-ethyl-phenyl)-*N*-(1-methoxylmethyl) ethylamine (II) was 98.2% and the enantiomeric excess 88.9% (*S*-isomer). The estimated reaction time, based on hydrogen uptake data, was 16 hours.

**Example 12**

2.3 mg of ligand (XVIII) and 1 mg of $[IrCODCl]_2$ (ligand:Ir ratio of 1.1:1) were pre-mixed in 1 ml of toluene under nitrogen and the mixture was stirred for 30 minutes. 10 μl of the previous mixture were fed to a reaction vial containing 7.7 mg $I_2$ ($I_2$:imine ratio of 0.005:1), 1.25 g of imine (I) (Ir:imine ratio of 1:200000) and 1.25 ml of toluene (toluene:imine ratio of 1:1 volume:weight), and the reaction vial was loaded into the hydrogenation equipment, which was purged with nitrogen. The reaction vessel was purged with $H_2$ in five cycles (20-30 bar, normal pressure) under stirring (650 rpm). The hydrogen pressure was set at 30 bar and the mixture heated to 70 °C for 16 hours under stirring (650 rpm). After cooling to room temperature the pressure was released, reaction mass was sampled (~10 μl, diluted to 1.5 ml with isopropanol) and analysed by HPLC. The conversion of the imine (I) to (*S*)-*N*-(2'-methyl-6'-ethyl-phenyl)-*N*-(1-methoxylmethyl) ethylamine (II) was 97.6% and the enantiomeric excess was 89.0% (*S*-isomer). The estimated reaction time, based on hydrogen uptake data, was 10 hours.

**Examples 13 to 15**

Using the procedure of example 12 with different additives, the following values of conversion and enantiomeric excess were obtained:

Table VI

| | Example 13 | Example 14 | Example 15 |
|---|---|---|---|
| LiI :imine ratio | 0.005:1 | 0.005:1 | --- |
| $I_2$:imine ratio | --- | --- | 0.0025:1 |
| Temperature (°C) | 70 | 60 | 70 |
| $H_2$ Pressure (bar) | 30 | 30 | 30 |
| Estimated reaction time (hours) | 5 | 28 | 8 |
| % conversion | 98.0 | 96.0 | 98.0 |
| % enantiomeric excess | 88.7 | 89.5 | 88.7 |

**Example 16**

2.3 mg of ligand (XVIII) and 1 mg of $[IrCODCl]_2$ (ligand:Ir ratio of 1.1:1) were pre-mixed in 1 ml of toluene under nitrogen and the mixture was stirred for 30 minutes. 32 µl of the previous mixture were fed to a reaction vessel containing 15.5 mg $I_2$ ($I_2$:imine ratio of 0.0025:1), 5 g of imine (I) (Ir:imine ratio of 1:250000) and 5 ml of toluene (toluene:imine ratio 1:1 volume:weight), and the vessel was purged with nitrogen. The reaction vessel was purged with $H_2$ in three cycles (10-20 bar, normal pressure) under stirring (750 rpm). The hydrogen pressure was set at 30 bar and the mixture heated to 70 °C for 20 hours under stirring (750 rpm). After cooling to room temperature the pressure was released, reaction mass was sampled (~10 µl, diluted to 1.5 ml with isopropanol) and analysed by HPLC. The conversion of the imine (I) to (*S*)-*N*-(2'-methyl-6'-ethyl-phenyl)-*N*-(1-methoxylmethyl) ethylamine (II) was 98.9% and the enantiomeric excess 88.2% (*S*-isomer). The estimated reaction time, based on hydrogen uptake data, was 18 hours.

**Comparative example**

1.9 mg of (R,$S_P$)-Xyliphos (III) and 1 mg of $[IrCODCl]_2$ were pre-mixed in 1 ml of toluene under nitrogen and the mixture stirred for 20-30 minutes. 32 µl of the previous mixture were fed to a reaction vessel containing 12.4 mg $I_2$, 216 mg acetic acid, 2.5 g of imine (I) and 10 ml of toluene, and the reaction vessel was purged with nitrogen. The reaction vessel was purged with $H_2$ in three cycles (10-20 bar, normal pressure) under stirring. The hydrogen pressure was set at 30 bar and the mixture heated to 70 °C for 18 hours under stirring. After cooling to room temperature the pressure was released, reaction mass was sampled (~10 µl, diluted to 1.5 ml with isopropanol) and analysed by HPLC. The conversion of the imine (I) to (*S*)-*N*-(2'-methyl-6'-ethyl-phenyl)-*N*-(1-methoxylmethyl) ethylamine (II) was 93.2% and the enantiomeric excess 74.6% (*S*-isomer). The estimated reaction time, based on hydrogen uptake data, was 12 hours.

2.2 mg of ligand (XVIII) and 1 mg of $[IrCODCl]_2$ were pre-mixed in 1 ml of toluene under nitrogen and the mixture stirred for 20-30 minutes. 32 µl of the previous mixture were fed to a reaction vessel containing 12.4 mg $I_2$, 2.5 g of imine (I) and 10 ml of toluene, and the reaction vessel was purged with nitrogen. The reaction vessel was purged with $H_2$ in three cycles (10-20 bar, normal pressure) under stirring. The hydrogen pressure was set at 30 bar and the mixture heated to 70 °C for 18 hours under stirring. After cooling to room temperature the pressure was released, reaction mass was sampled (~10 µl, diluted to 1.5 ml with isopropanol) and analysed by HPLC. The conversion of the imine (I) to (*S*)-*N*-(2'-methyl-6'-ethyl-phenyl)-*N*-(1-methoxylmethyl) ethylamine (II) was 98.8% and the enantiomeric excess 91.1% (*S*-isomer). The estimated reaction time, based on hydrogen uptake data, was 2 hours.

The results of the comparative test show that the process according to the invention results in a 5% increase conversion, a 16% increase in selectivity, and a reduction in reaction time from 12 to 2 hours when compared to the process using xyliphos.

It can be seen that the process according to the invention corresponds to a significant improvement over prior art since it was possible to operate at a imine to metal ratio of 250000:1 and achieve a conversion of 98.9% and an enantiomeric excess of 88.2% in a reaction time of 18 hours.

## Claims

1. A process of production of S-metolachlor comprising a step of hydrogenation of *N*-(2'-methyl-6'-ethyl-phenyl)-*N*-(1-methoxylmethyl)ethylimine to (S)-N-(2'-methyl-6'-ethylphenyl)-*N*-(1-methoxylmethyl)ethylamine, **characterized in that** *N*-(2'-methyl-6'-ethyl-phenyl)-*N*-(1-methoxylmethyl) ethylimine contacts with hydrogen in the presence of:

    - a coordination compound formed by a ligand having the structural formula

    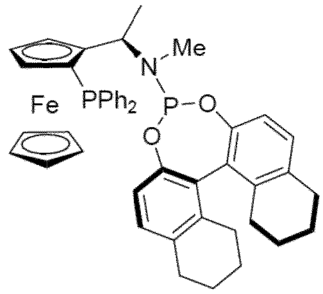

    (*R*)-*N*-[(*R*),(*R*)-5,5',6,6',7,7',8,8'-octahydro-1,1'-binaphthyl-2,2'-dioxyphosphino]-*N*-methyl-1-[(*S*)-2-(diphenylphosphino) ferrocenyl]ethylamine
    or

    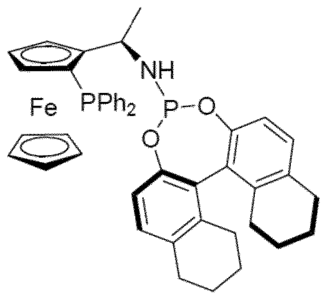

    (*R*)-*N*-[(*R*),(*R*)-5,5',6,6',7,7',8,8'-octahydro-1,1'-binaphthyl-2,2'-dioxyphosphino]-1-[(*S*)-2-(diphenylphosphino)ferrocenyl]ethylamine,
    and a diiridium complex of the formula $[IrXCl]_2$, wherein X is 1,5-cyclooctadiene (COD), at a molar ratio of ligand : Iridium in the range of 1:2 to 2:1;
    - an additive selected from the group consisting of iodine or an alkaline metal salt thereof; and
    - an aprotic solvent,
    at a temperature in the range of 20 °C to 90 °C and a hydrogen pressure in the range of 5 to 80 bar.

2. The process of claim 1 wherein the molar ratio of ligand:Iridium is 1.1:1.

3. The process of claim 1 wherein the molar ratio between *N*-(2'-methyl-6'-ethyl-phenyl)-*N*-(1-methoxylmethyl) ethylimine and the ligand is in the range of 100:1 to 300000:1.

4. The process of claim 3 wherein the molar ratio between *N*-(2'-methyl-6'-ethyl-phenyl)-*N*-(1-methoxylmethyl) ethylimine and the ligand is in the range of 100000:1 to 250000:1.

5. The process of claim 1 wherein the molar ratio between the additive and *N*-(2'-methyl-6'-ethyl-phenyl)-*N*-(1-methoxylmethyl)ethylamine is in the range of 0.00125:1 to 0.050:1.

6. The process of claim 5 wherein the molar ratio between the additive and *N*-(2'-methyl-6'-ethyl-phenyl)-*N*-(1-methoxylmethyl)ethylamine is in the range of 0.0025:1 to 0.0050:1.

7. The process of claim 1 wherein the additive is iodine or lithium iodide.

**8.** The process of claim 1 wherein the aprotic solvent is selected from the group consisting of dichloromethane, toluene, xylene and n-heptane.

**9.** The process of claim 8 wherein the aprotic solvent is toluene.

**10.** The process of claim 1 wherein the ratio between the aprotic solvent and *N*-(2'-methyl-6'-ethyl-phenyl)-N-(1-methoxylmethyl)ethylamine is in the range of 1:2 volume:weight to 150:1 volume:weight.

**11.** The process of claim 10 wherein the ratio between the aprotic solvent and N-(2'-methyl-6'-ethyl-phenyl)-N-(1-methoxylmethyl)ethylamine is in the range of 1:1 volume:weight to 4:1 volume/weight.

**12.** The process of claim 1 wherein the hydrogen pressure is in the range of 20 to 80 bar.

**13.** The process of claim 12 wherein the hydrogen pressure is 30 bar.

**14.** The process of claim 1 wherein the reaction is carried out at a temperature of 50 °C to 70 °C.

**15.** The process of claim 14 wherein the reaction is carried out at a temperature of 70 °C.

**Patentansprüche**

**1.** Verfahren zur Herstellung von *S*-metolachlor in dem eine Hydrierung von *N*-(2'-methyl-6'-ethyl-phenyl)-*N*-(1-methoxylmethyl)ethylamine zu (*S*)-*N*-(2'-methyl-6'-ethylphenyl)-*N*-(1-methoxylmethyl)ethylamin vorkommt, die gekennzeichnet davon ist dass *N*-(2'-methyl-6'-ethyl-phenyl)-*N*-(1-methoxylmethyl) ethylamin in Kontakt mit Wasserstoff kommt in Anwesenheit von:

- einer Koordinationsverbindung bestehend aus einem liganden mit der hier unten dargestelten Stukturellen form

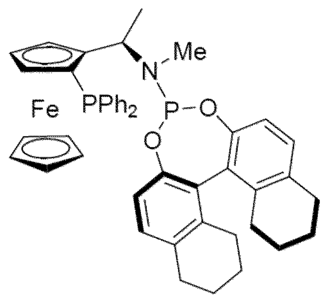

(*R*)-*N*-[(*R*),(*R*)-5,5',6,6',7,7',8,8'-octahydro-1,1'-binaphthyl-2,2'-dioxyphosphino]-*N*-methyl-1-[(*S*)-2-(diphenylphosphino) ferrocenyl]ethylamin
oder

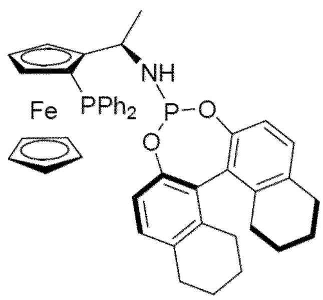

(*R*)-*N*-[(*R*),(*R*)-5,5',6,6',7,7',8,8'-octahydro-1,1'-binaphthyl-2,2'-dioxyphosphino]-1-[(*S*)-2-(diphenylphosphino)ferrocenyl]ethylamin, und ein diiridium komplex mit der Formel $[IrXCl]_2$, worin X ein 1,5-cyclooctadiene (COD) ist, indem das Molarverhältnis vom liganden : Iridium im bereich von 1:2 bis zu 2:1 liegt;

- ein Zusatzstoff ausgewählt von einer Gruppe bestehend aus Jod oder einem davon alkalischen Metallsalz; und
- ein aprotisches lösungsmittel,

bei einer Temperatur zwischen 20°C und 90°C und einem Wasserstoffdruck im Bereich von 5 bis zu 80 bar.

**2.** Verfahren nach Anspruch 1 worin das Molarverhältnis von ligand:Iridium 1.1:1 ist.

**3.** Verfahren nach Anspruch 1 worin das Molarverhältnis zwischen *N*-(2'-methyl-6'-ethyl-phenyl)-*N*-(1-methoxylmethyl) ethylimin und dem liganden im Bereich von 100:1 bis zu 300000:1 liegt.

**4.** Verfahren nach Anspruch 3 worin das Molarverhältnis zwischen *N*-(2'-methyl-6'-ethyl-phenyl)-*N*-(1-methoxylmethyl) ethylimin und dem liganden im Bereich von 100000:1 bis zu 250000:1 liegt.

**5.** Verfahren nach Anspruch 1 worin das Molarverhältnis zwischen dem Zustatzstoff und *N*-(2'-methyl-6'-ethyl-phenyl)-*N*-(1-methoxylmethyl)ethylamin im Bereich von 0.00125:1 bis zu 0.050:1 liegt.

**6.** Verfahren nach Anspruch 5 worin das Molarverhältnis zwischen dem Zustatzstoff und *N*-(2'-methyl-6'-ethyl-phenyl)-*N*-(1-methoxylmethyl)ethylamin im Bereich von 0.0025:1 to 0.0050:1 liegt.

**7.** Verfahren nach Anspruch 1 worin der Zustatzstoff Jod oder Lithiumiodid ist.

**8.** Verfahren nach Anspruch 1 worin das aprotische Lösungsmittel aus einer Gruppe ausgewählt wird bestehend aus Dichlormethan, Toluol, Xylol und n-Heptan.

**9.** Verfahren nach Anspruch 8 worin das aprotische Lösungsmittel Toluol ist.

**10.** Verfahren nach Anspruch 1 worin das Verhältnis zwischen das aprotische Lösungsmittel und N-(2'-methyl-6'-ethyl-phenyl)-N-(1-methoxylmethyl)ethylamin im Bereich von 1:2 Volumen:Gewicht bis zu 150:1 Volumen:Gewicht liegt.

**11.** Verfahren nach Anspruch 10 worin das Verhältnis zwischen das aprotische Lösungsmittel und N-(2'-methyl-6'-ethylphenyl)-N-(1-methoxylmethyl)ethylamine im Bereich von 1:1 Volumen:Gewicht bis zu 4:1 Volumen:Gewicht liegt.

**12.** Verfahren nach Anspruch 1 worin der Wasserstoffdruck im Bereich von 20 bis 80 bar liegt.

**13.** Verfahren nach Anspruch 12 worin der Wasserstoffdruck bei 30 bar liegt.

**14.** Verfahren nach Anspruch 1 worin die Reaktion bei einer Temperatur von 50°C bis zu 70°C ausgeführt wird.

**15.** Verfahren nach Anspruch 14 worin die Reaktion bei einer Temperatur von 70°C ausgeführt wird.

## Revendications

**1.** Procédé de production de S-métolachlore comprenant une étape d'hydrogénation de la *N*-(2'-méthyl-6'-éthyl-phényl)-*N*-(1-méthoxyéthyle)éthylamine en (*S*)-*N*-(*2'-méthyl-6'*-éthyl-phényl)-*N*-(1-méthoxyéthyle)éthylamine, **caractérisé en ce que** la *N*-(2'-méthyl-6'-éthyl-phényl)-*N*-(1-méthoxyéthyle)éthylamine est mise en contact avec l'hydrogène en présence de :

- un composé de coordination formé par un ligand ayant la formule structurelle

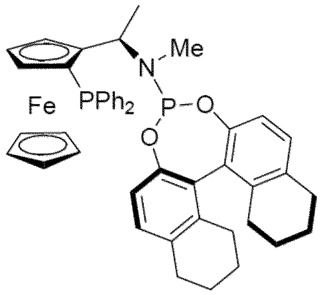

(*R*)-*N*-[(*R*),(*R*)-5,5',6,6',7,7',8,8'-octahydro-1,1'-binaphtyle-2,2'-dioxyphosphino]-*N*-*méthyl-1*-[(*S*)-2-(diphénylphosphino) ferrocenyl]éthylamine
ou

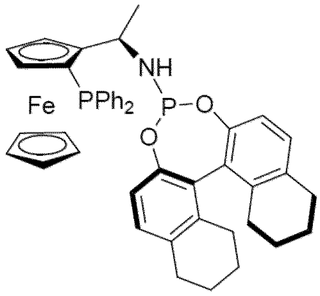

(*R*)-*N*-[(*R*),(*R*)-5,5',6,6',7,7',8,8'-octahydro-1,1'-binaphtyle-2,2'-dioxyphosphino]-1-[(*S*)-2-(diphénylphosphino)ferrocenyl]éthylamine,
et un complexe de diiridium de formule $[IrXCl]_2$, dans lequel X est le 1,5-cyclooctadiène (COD), à un rapport molaire ligand:iridium dans la gamme de 1:2 à 2:1 ;
- un additif choisi parmi le groupe consistant en l'iode et un sel de métal alcalin de celui-ci ; et
- un solvant aprotique,
à une température comprise entre 20°C et 90°C et à une pression d'hydrogène comprise entre 5 et 80 bar.

**2.** Procédé selon la revendication 1, dans lequel le rapport molaire ligand:Iridium est de 1,1:1.

**3.** Procédé selon la revendication 1, dans lequel le rapport molaire entre la *N*-(2'-méthyl-6'-éthyl-phényl)-*N*-(1-méthoxyéthyle) éthylamine et le ligand est situé dans la plage de 100:1 à 300000:1.

**4.** Procédé selon la revendication 3, dans lequel le rapport molaire entre la *N*-(2'-méthyl-6'-éthyl-phényl)-*N*-(1-méthoxyéthyle) éthylamine et le ligand est situé dans la plage de 100000:1 à 250000:1.

**5.** Procédé selon la revendication 1, dans lequel le rapport molaire entre l'additif et la *N*-(2'-méthyl-6'-éthyl-phényl)-*N*-(1-méthoxyéthyle)éthylamine est situé dans la plage de 0,00125:1 à 0,050:1.

**6.** Procédé selon la revendication 5, dans lequel le rapport molaire entre l'additif et la *N*-(2'-méthyl-6'-éthyl-phényl)-*N*-(1-méthoxyéthyle)éthylamine est situé dans la plage de 0,0025:1 à 0,0050:1.

**7.** Procédé selon la revendication 1 dans lequel l'additif est l'iode ou l'iodure de lithium.

**8.** Procédé selon la revendication 1, dans lequel le solvant aprotique est choisi parmi le groupe consistant en le dichlorométhane, le toluène, le xylène et le n-heptane.

**9.** Procédé selon la revendication 8 dans lequel le solvant aprotique est le toluène.

**10.** Procédé selon la revendication 1, dans lequel le rapport entre le solvant aprotique et la N-(2'-méthyl-6'-éthyl-phényl)-N-(1-méthoxyéthyle)éthylamine est compris entre 1:2 volume:poids et 150:1 volume:poids.

**11.** Procédé selon la revendication 10, dans lequel le rapport entre le solvant aprotique et la N-(2'-méthyl-6'-éthyl-phényl)-N-(1-méthoxyéthyle)éthylamine est situé dans la plage de 1:1 volume:poids à 4:1 volume/poids.

**12.** Procédé selon la revendication 1, dans lequel la pression de l'hydrogène est comprise entre 20 et 80 bar.

**13.** Procédé selon la revendication 12 dans lequel la pression de l'hydrogène est de 30 bar.

**14.** Procédé selon la revendication 1, dans lequel la réaction est effectuée à une température de 50°C à 70°C.

**15.** Procédé selon la revendication 14 dans lequel la réaction est effectuée à une température de 70°C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9521151 A
- US 6525210 B
- WO 2010094164 A
- CN 101857612
- WO 2012116493 A
- WO 2006012045 A1


### Non-patent literature cited in the description


- **HU, XIANG-PING et al.** *Organic Letters,* 2012, vol. 14 (13), 3554-3557
- **HU, XIANG-PING et al.** *Organic Letters,* 2004, vol. 6 (20), 3585-3588